# EUROPEAN PATENT SPECIFICATION

(11) **EP 3 917 534 B1**
(45) Date of publication and mention of the grant of the patent: **03.07.2024**
(21) Application number: 20708913.7
(22) Date of filing: 30.01.2020
(51) Int. Cl.: A61K 31/56, A61P 31/10

(54) **TRITERPENOID ANTIFUNGALS FOR THE TREATMENT OF FUNGAL OSTEO-ARTICULAR INFECTIONS**
TRITERPEN ANTIMYKOTISCHE MITTEL ZUR BEHANDLUNG VON OSTEO-ARTIKULÄREN MYKOTISCHEN INFEKTIONEN
COMPOSÉS ANTIMYCOTIQUES À BASE DE TRITERPÈNES POUR LE TRAITEMENT D'INFECTIONS FUNGALES OSTÉO-ARTICULAIRES

(30) Priority: 30.01.2019 US 201962798743 P
(43) Date of publication of application: 08.12.2021
(73) Proprietor: Scynexis, Inc., Jersey City, NJ 07302 (US)
(72) Inventor: ANGULO GONZALEZ, David A., Palmetto Bay, Florida 33157 (US)
(74) Representative: Harris, Jennifer Lucy
(86) International application number: PCT/US2020/015948
(87) International publication number: WO 2020/160316

(56) References cited:
- WO-A1-2010/019203
- STEPHEN A. WRING ET AL: "Preclinical Pharmacokinetics and Pharmacodynamic Target of SCY-078, a First-in-Class Orally Active Antifungal Glucan Synthesis Inhibitor, in Murine Models of Disseminated Candidiasis", ANTIMICROBIAL AGENTS AND CHEMOTHERAPY, vol. 61, no. 4, 30 January 2017 (2017-01-30), XP055518287, US ISSN: 0066-4804, DOI: 10.1128/AAC.02068-16
- Scynexis: "SCYNEXIS Announces Complete Results from Two Phase 2 Studies of Oral SCY- 078 in Patients with Candida spp. Infections and Closing of a $15 Million Term Loan", N.J.GLOBE NEWSWIRE, 5 October 2016 (2016-10-05), XP055518278, Retrieved from the Internet: URL:https://content.equisolve.net/scynexis /news/2016-10-05_SCYNEXIS_Announces_Comple te_Results_from_Two_85.pdf [retrieved on 2018-10-23]
- LAMOTH FREDERIC ET AL: "Antifungal Activities of SCY-078 (MK-3118) and Standard Antifungal Agents against Clinical Non-Aspergillus Mold Isolates", ANTIMICROBIAL AGENTS AND CHEMOTHERAPY, vol. 59, no. 7, July 2015 (2015-07), pages 4308-4311, XP002798729,
- DATABASE BIOSIS [Online] BIOSCIENCES INFORMATION SERVICE, PHILADELPHIA, PA, US; July 2015 (2015-07), LAMOTH FREDERIC ET AL: "Antifungal Activities of SCY-078 (MK-3118) and Standard Antifungal Agents against Clinical Non-Aspergillus Mold Isolates", Database accession no. PREV201500708401 & ANTIMICROBIAL AGENTS AND CHEMOTHERAPY, vol. 59, no. 7, July 2015 (2015-07), pages 4308-4311, ISSN: 0066-4804(print), DOI: 10.1128/AAC.00234-15

## Description

### FIELD OF THE INVENTION

The present invention relates to the use of enfumafungin derivative triterpenoid antifungal compounds to treat fungal infections of the bone (including osteomyelitis) and fungal infection of associated connective tissues such as periosteum and other osteo and articular tissues including but not limited to bone marrow, joint fluid, intervertebral discs and other cartilage structures, etc., collectively referred to in this disclosure as "osteo-articular infections." More particularly, the invention relates to the use of enfumafungin derivative triterpenoids (or pharmaceutically acceptable salts or hydrates thereof) that are inhibitors of (1,3)-β-D-glucan synthesis, in the treatment of fungal infections (such as yeast or mold infections) that occur in osteo-articular structures and for which long-term antifungal therapy (more than 4 weeks) and sufficient antifungal penetration into the affected tissues are needed, for resolution of the infection. Inhibitors of (1,3)-β-D-glucan synthesis are considered the treatment of choice for multiple fungal infections, particularly those cause by *Candida* spp., but currently available (1,3)-β-D-glucan synthase inhibitors (echinocandins) can only be administered intravenously and have limited penetration into osteo-articular structures. In accordance with the present invention, an (1,3)-β-D-glucan synthase inhibitor that shows enhanced penetration into osteo-articular structures and can be administered orally would provide an advantage in the treatment of fungal osteo-articular infections such as osteomyelitis, spondylodiscitis, and arthritis, to mention some, that typically require antifungal treatment for several months and for which currently available treatment options are not optimal due to limited penetration into osteo-articular structures and/or lack of availability of oral formulations for (1,3)-β-D-glucan synthase inhibitors.

### BACKGROUND OF THE INVENTION

Fungal infections are a major healthcare problem and are most commonly manifested as invasive or systemic fungal disease (*e.g*., candidemia, invasive aspergillosis), localized fungal infections (*e.g*., bone, pleural empyema, abscess localized in abdomen, brain, lung, etc.) and mucocutaneous infections (*e.g*., oral, esophageal and vulvovaginal candidiasis). The type and scope of the infection depends on the virulence factors of the fungal pathogen, the host's defenses, and the anatomic areas involved.

Severe systemic or invasive fungal infections are more common in immune-compromised patients such as patients receiving chemotherapy to treat malignancies, or receiving immunomodulatory agents to treat chronic inflammatory conditions, or suffering from immune deficiencies, either acquired or due to genetic disorders. Despite currently available antifungal therapies, systemic fungal infections are associated with a mortality rate of up to 50%, depending on the pathogen and the underlying condition of the patient. Localized fungal infections typically originate via dissemination of fungi (such as yeast) from a local area where they normally colonize to an area that is normally sterile (*e.g*., abscess in abdominal cavity after gut perforation or surgery) or from fungi entering the blood or lymphatic system that reaches a particular organ (*e.g*., osteo-articular structures, lung, liver, spleen) and develops a deep seated infection.

Fungal osteo-articular infections may follow either hematogenous dissemination, mostly in immunocompromised hosts, or skin infection with local dissemination. While they used to be relatively rare, the incidence of fungal osteo-articular infections is increasing with an increase in the prevalence of factors predisposing to invasive fungal disease, such as the use of central venous catheters, broad spectrum antibiotics, immunosuppression, and complex surgeries. Definitive diagnosis relies on bone or synovial culture or biopsy. The most common cause of osteo-articular infections in developed counties is *Candida* spp. followed by *Aspergillus* spp., both of which are ubiquitous fungi. Other fungi reported as causing osteo-articular infection may cause such infection only in limited geographic areas (*Histoplasmosis* spp., *Blastomyces* spp., *Coccidioides* spp., *Sporothrix* spp.) or in areas with high incidence of co-morbid conditions such as AIDS (*e.g*., *Cryptococcus* spp.); however, many other yeast, molds, and dimorphic fungi have the ability to infect osteo-articular structures. Almost all osteo-articular infections require more than four weeks of treatment.

Most patients with osteomyelitis present with a subacute to chronic course. Involvement of two or more bones is common, and therefore, when a single focus of infection is identified, there should be a search for other sites of involvement. The axial skeleton, especially the spine, is the most common site of involvement in adults; in children, the long bones are more commonly involved. These infections may be difficult to diagnose and eradicate. Current treatment guidelines indicate that historically, Amphotericin B deoxycholate (only available intravenously) has been the most commonly used agent. (Clinical Practice Guideline for the Management of Candidiasis: 2016 Update by the Infectious Diseases Society of America. Pappas PG, Kauffman CA, Andes DR, Clancy CJ, Marr KA, Ostrosky-Zeichner L, Reboli AC, Schuster MG, Vazquez JA, Walsh TJ, Zaoutis TE, Sobel JD. Clin Infect Dis. 2016 Feb 15;62(4):e1-50. doi: 10.1093/cid/civ933. Epub 2015 Dec 16.) Recent literature favors the use of azoles or an echinocandin over Amphotericin B. Fluconazole has been used successfully as initial therapy for patients who have susceptible isolates, but treatment failures have been reported. Fluconazole is not active against *Aspergillus* spp., and the increased incidence of azole-resistant *Candida* strains poses a challenge to physicians to select a treatment that can be administered for a long-term period and has adequate antifungal spectrum of activity to cover the causative pathogen. Cure rates appear to be significantly higher when an antifungal agent is administered for at least 6 months. Surgical debridement is frequently performed in conjunction with antifungal therapy.

More generally, all clinically relevant classes of antifungals available for use to date such as azoles, polyenes, and echinocandins, have shown limitations for the treatment of osteo-articular fungal infections due to limited distribution into the affected tissues (echinocandins), emergence of resistance (azoles), lack of availability of oral formulations (systemic polyenes and echinocandins), significant toxicity or drug-drug interaction potential (azoles and polyenes). There is a need in the art for antifungal therapy for humans, particularly in the treatment of fungal infections occurring in osteo-articular structures for which limited orally available therapeutic options exist.

For an antifungal to effectively treat an infection, it must achieve efficacious concentrations into the affected tissues. However, drug penetration into bone is limited for most conventional antifungal agents. The concentration of 18F-fluconazole in bone is approximately 33% of the plasma concentration in humans (and 100% in rabbits), and anidulafungin (echinocandin) concentrations in the bone of neonatal rats after a single dose are less than those in plasma, with a bone/plasma concentration ratio of 0.21. Human bone penetration data for other echinocandins (caspofungin and micafungin) is limited; however, skeletal muscle concentrations of anidulafungin in rats are comparable to those in plasma, whereas for caspofungin, skeletal muscle concentrations in mice are less than 50% of those in plasma. These observations are in line with the low volume of distribution reported for the three echinocandins, ranging from 0.15 to 0.8 liters/kg, indicating that tissue concentrations higher than in plasma, are not expected. (Tissue penetration of antifungal agents. Felton T, Troke PF, Hope WW. Clin Microbiol Rev. 2014 Jan;27(1):68-88. doi: 10.1128/CMR.00046-13. Review.)

The ability of a pathogen to form biofilms has been associated with osteo-articular infections. Although this association has so far been better characterized with respect to bacterial infections, the basic scientific principals likely may apply to fungal infections as well. An important factor in the development of the chronic form of osteo-articular infections may be the ability of the pathogen to form a biofilm. A biofilm is a microbially derived sessile community, typified by cells that are attached to a substratum, interface, or to each other, are embedded in a matrix of extracellular polymeric substance, and exhibit an altered phenotype with regard to growth, gene expression, and protein production. Biofilm formation allows for immune evasion as well as resistance of antimicrobial agents, such that the only way to successfully treat such infections is to remove the diseased tissue. Therapeutics that are able to target formed biofilm could provide an advantage in the treatment of osteo-articular infections over those that do not affect this phenomenon.

Enfumafungin is a hemiacetal triterpene glycoside that is produced in fermentations of a *Hormonema* spp. associated with living leaves of *Juniperus communis* (U.S. Pat. No. 5,756,472; Pelaez et al., Systematic and Applied Microbiology, 23:333-343 (2000); Schwartz et al., JACS, 122: 4882-4886 (2000); Schwartz, R.E., Expert Opinion on Therapeutic Patents, 11(11): 1761-1772 (2001)). Enfumafungin is one of the several triterpene glycosides that have *in vitro* antifungal activities. The mode of the antifungal action of enfumafungin and other antifungal triterpenoid glycosides was determined to be the inhibition of fungal cell wall glucan synthesis by their specific action on (1,3)-β-D-glucan synthase (Onishi et al., Antimicrobial Agents and Chemotherapy, 44: 368-377 (2000); Pelaez *et al.,* (2000)). 1,3-β-D-glucan synthase remains an attractive target for antifungal drug action because it is present in many pathogenic fungi and therefore affords a broad antifungal spectrum. In addition, because there is no mammalian counterpart to (1,3)-β-D-glucan synthase, the enfumafungin derivatives described herein have little or no mechanism-based toxicity. The triterpenoid compound derivatives of enfumafungin used according to this invention have demonstrated activity against fungal isolates of *Candida* spp., including those isolates that are resistant to azoles or other glucan synthase inhibitors (*e.g*., lipopeptides agents such echinocandins), indicating that the biological and molecular target of the enfumafungin derivatives is different from that of other glucan synthase inhibitors.

Various enfumafungin derivatives have been disclosed, e.g., in International Patent Publication Nos. WO 2007/126900, WO 2007/127012 and WO 2010/019203.

Certain representatives of these enfumafungin derivatives can be administered orally and have shown antifungal activity against fungal species commonly implicated in osteo-articular infections. At the same time, however, previous reports have reported limited penetration into bone of other inhibitors of (1,3)-β-D-glucan synthesis.

The anti-fungal activity and ability of SCY-078, a representative compound of enfumafungin derivatives described herein, to treat an osteo-articular fungal infection was evaluated in several feasibility studies.

As a first step of the feasibility evaluation, the tissue distribution of SCY-078 was evaluated in an animal model. (Wring S, Borroto-Esoda K, Solon E, Angulo D, SCY-078, a Novel Fungicidal Agent, Demonstrates Distribution to Tissues Associated with Fungal Infections during Mass Balance Studies with Intravenous and Oral [14C]SCY-078 in Albino and Pigmented Rats, Antimicrob Agents Chemother. 2019 Jan 29; 63(2). pii: e02119-18. doi: 10.1128 / AAC.02119-18. Print 2019 Feb.) Male albino Wistar Han (WH; Charles River, Raleigh, NC) (n=38) or male (n =18) and female (n=3) pigmented Long-Evans (LE; Hilltop Lab Animals, Inc., Scottdale, PA) rats received [14C]SCY-078 by oral administration (15 mg/kg, ~150 µCi/kg, in aqueous 0.5% methylcellulose) or i.v. administration (5 mg/kg, ~108 µCi/kg, 7.5:1 molar ratio of Captisol:SCY-078 in saline) as a 1-h infusion (10 ml/kg/h). WH rats were used for mass balance and pharmacokinetic (PK) determinations after i.v. and oral doses, and both WH and LE rats were used for Quantitative whole-body autoradiography (QWBA) determinations. Dose levels were selected to reflect the clinically relevant 11.2-µg·h/ml target exposure for *Candida* spp. infections. The concentration, homogeneity, radio purity, and stability of dosing formulations were confirmed to be acceptable before dosing. For QWBA whole-body sections (~40 µm thick via Leica CM3600 cryomicrotome; Nussloch, Germany), where all major tissues, organs, and biological fluids were represented, sections were exposed for phosphor imaging (Fuji Biomedical, Stamford, CT) together with calibration standards. Animals were deeply anesthetized with isoflurane anesthesia and, after blood samples were obtained, were euthanized by freezing in a hexane/solid carbon dioxide bath for at least 15 min. The imaging plate was scanned with the GE Healthcare Typhoon FLA 9500 image acquisition system (GE/Molecular Dynamics, Sunnyvale, CA). Quantification was performed by image densitometry with MCID image analysis software (v. 7.0; Interfocus Imaging Ltd., Linton, Cambridge, UK), and a standard curve was constructed from the integrated response (molecular dynamics counts [MDC]/mm2) and the nominal concentrations of the 14C-calibration standards. The concentrations of radioactivity were expressed as [14C]SCY-078 µg equiv/g tissue. The lower limit of quantitation was 0.024 and 0.049 µg equiv/g of tissue for i.v. and oral doses of SCY-078, respectively.

Tissue to blood AUC ratios of total radioactivity after 15 mg/kg oral dose of [¹⁴C]-SCY-078 to male pigmented Long-Evans rats are illustrated in the table below:

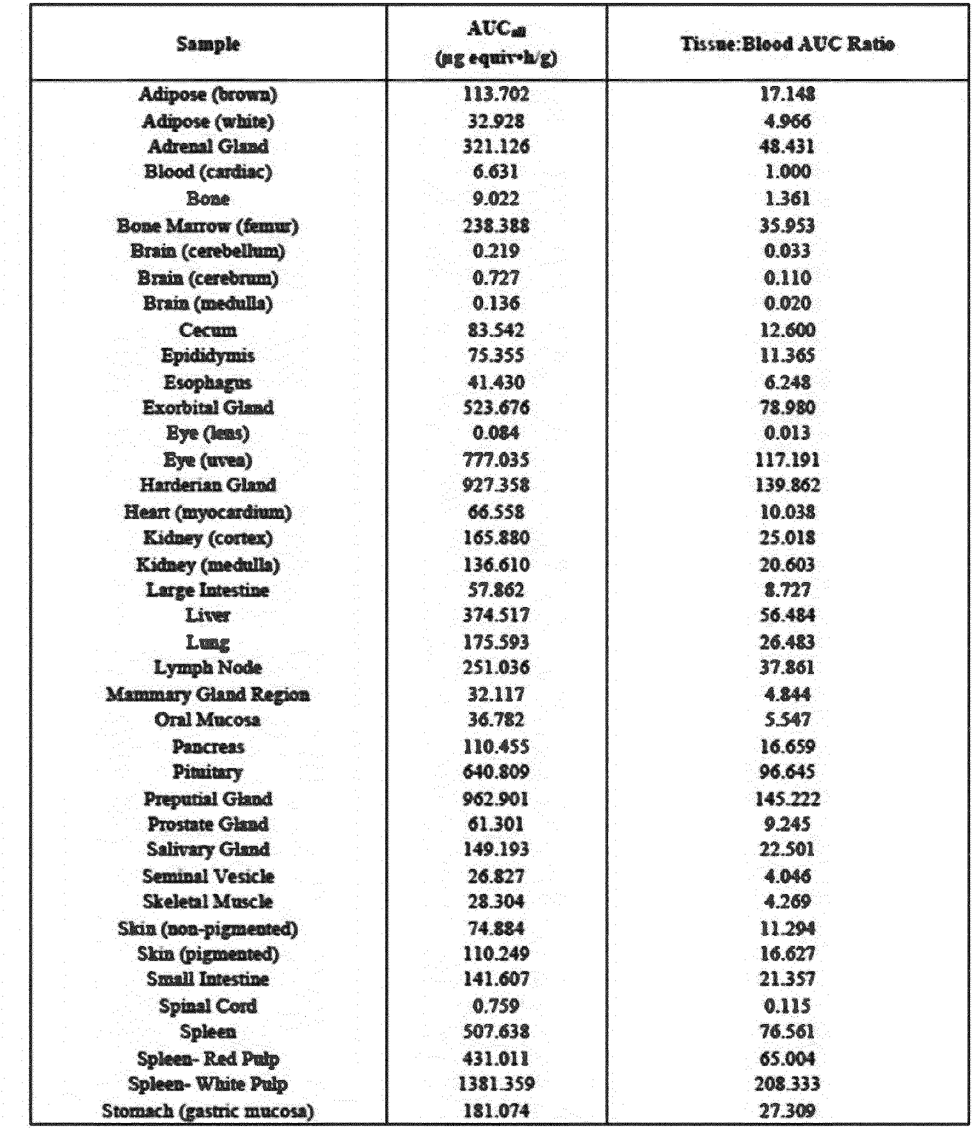

The exposure observed in bone and bone marrow exceeded the exposure measured in plasma in this study.

The antifungal activity of SCY-078 against the planktonic and sessile (biofilm) forms of 178 *Candida* and non*-Candida* isolates causing fungaemia in patients recently admitted to a large European hospital was previously evaluated. (Marcos-Zambrano LJ, Gómez-Perosanz M, Escribano P, Bouza E, Guinea J, The novel oral glucan synthase inhibitor SCY-078 shows in vitro activity against sessile and planktonic Candida spp., J Antimicrob Chemother. 2017 Jul 1;72(7):1969-1976. doi: 10.1093/jac/dkx010.) The in vitro activity of SCY-078 against the planktonic form of the isolates was assessed using EUCAST E Def 7.3 and CLSI M27-A3. Antibiofilm activity was assessed using the XTT reduction assay. SCY-078 showed potent *in vitro* activity against *Candida* and non*-Candida* isolates. SCY-078 showed activity against the biofilms. These observations were confirmed by assessing biofilm structure by scanning electron microscopy after antifungal treatment. This study showed high *in vitro* activity of SCY-078 against invasive *Candida* isolates in both sessile and planktonic forms. Notably, in comparison, azoles (the only orally available antifungal agents indicated for the treatment of osteo-articular fungal infections) are generally known not to have anti-biofilm activity.

The activity of SCY-078 in murine models of disseminated candidiasis is described in Wring et al, Preclinical Pharmacokinetics and Pharmacodynamic Target of SCT-078, a First-in-Class orally active antifungal glucan synthesis inhibitor, in murine models of disseminated candidiasis, Antimicrob Agents Chemother. 2017; 61(4).

### SUMMARY OF THE INVENTION

The enfumafungin derived triterpenoid compound SCY-078-a representative compound of enfumafungin derivatives described herein-showed a high level of clinical efficacy in osteo-articular fungal infections.

The compound unexpectedly demonstrated enhanced penetration into osteo-articular structures when compared to other (1,3)-β-D-glucan synthase inhibitors. SCY-078 can be administered orally, which provides an advantage in the treatment of fungal osteo-articular infections such as osteomyelitis, spondylodiscitis, arthritis, to mention some, that typically require antifungal treatment for several months.

The present invention relates to using enfumafungin derivatives for the treatment of fungal infections that occur in osteo-articular structures. Enfumafungin derivatives, and pharmaceutically acceptable salts or hydrates thereof, are useful in the inhibition of (1,3)-β-D-glucan synthase, and are particularly useful in treatment of fungal infections that occur in osteo-articular structures, which are infection situations where potent antifungal activity is needed in the art.

The present invention addresses needs in the art such as those described above because the enfumafungin derivatives described herein (a) unexpectedly achieved high tissue penetration into bone, (b) showed surprising clinical efficacy in difficult to treat osteo-articular fungal infections, (c) can be administered orally allowing for optimal therapy in these infections that often require several months of therapy, and (d) showed activity against biofilms which may enhance the ability to successfully treat these chronic infections.

Applications of this invention include but are not limited to the ability to more easily achieve a successful outcome in the treatment of osteo-articular infections because of the reasons outlined above.

The present invention provides a compound of Formula (II): 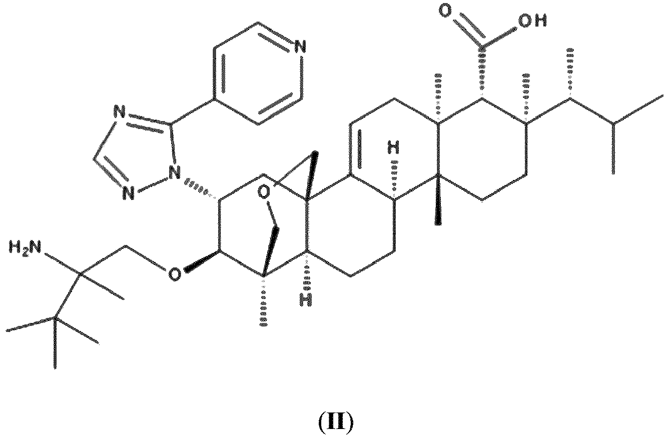 which is (1*S*,4a*R*,6a*S*,7*R*,8*R*,10a*R*,10b*R*,12a*R*,14*R*,15*R*)-15-[[2-amino-2,3,3-trimethylbutyl]oxy]-8-[(1*R*)-1,2-dimethylpropyl]-14-[5-(4-pyridinyl)-1*H*-1,2,4-triazol-1-yl]-1,6,6a,7,8,9,10,10a,10b,11,12,12a-dodecahydro-1,6a,8,10a-tetramethyl-*4H*-1,4a-propano*-2H-*phenanthro[1,2-c]pyran-7-carboxylic acid, or a pharmaceutically acceptable salt or hydrate thereof, for use in a method of treating a fungal osteo-articular infection in a subject in need thereof, wherein the fungal osteo-articular infection is osteomyelitis, spondylodiscitis, or arthritis.

### DETAILED DESCRIPTION OF THE INVENTION

The present invention relates to enfumafungin derivative triterpenoid antifungal compounds for use in treating fungal infections of the bone (including osteomyelitis) and fungal infections of associated connective tissues such as periosteum and other osteo and articular tissues including but not limited to bone marrow, joint fluid, intervertebral discs and other cartilage structures, etc., collectively referred to in this disclosure as "osteo-articular infections." More particularly, the invention relates to enfumafungin derivative triterpenoids (or pharmaceutically acceptable salts or hydrates thereof) that are inhibitors of (1,3)-β-D-glucan synthesis, for use in the treatment of fungal infections that occur in osteo-articular structures and for which long-term antifungal therapy (more than 4 weeks) and sufficient antifungal penetration into the affected tissues are needed, for resolution of the infection. Inhibitors of (1,3)-β-D-glucan synthesis are considered the treatment of choice for multiple fungal infections, particularly those caused by *Candida* spp., but currently available (1,3)-P-D-glucan synthase inhibitors (echinocandins) can only be administered intravenously and have limited penetration into osteo-articular structures. In accordance with the present invention, an (1,3)-β-D-glucan synthase inhibitor that shows enhanced penetration into osteo-articular structures and can be administered orally provides an advantage in the treatment of fungal osteo-articular infections such as osteomyelitis, spondylodiscitis, and arthritis, to mention some, that typically require antifungal treatment for several months and for which currently available treatment options are not optimal due to limited penetration into osteo-articular structures and/or lack of availability of oral formulations for (1,3)-β-D-glucan synthase inhibitors.

The present invention provides a compound of Formula (II): 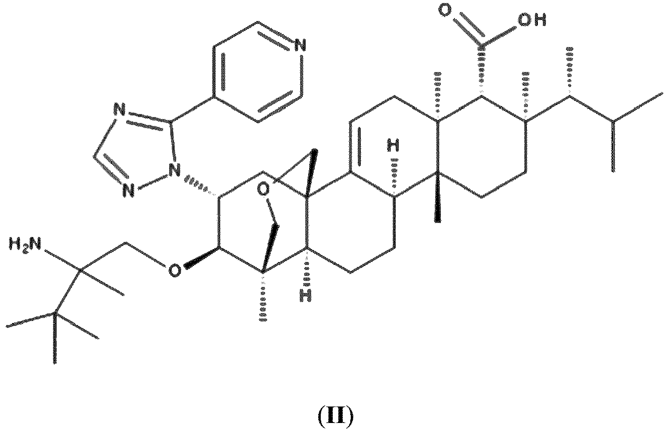 which is (1*S*,4a*R*,6a*S*,7*R,*8*R*,10a*R*,10b*R*,12a*R*,14*R*,15*R*)-15-[[2-amino-2,3,3-trimethylbutyl]oxy]-8-[(1*R*)-1,2-dimethylpropyl]-14-[5-(4-pyridinyl)-1*H*-1,2,4-triazol-1-yl]-1,6,6a,7,8,9,10,10a,10b,11,12,12a-dodecahydro-1,6a,8,10a-tetramethyl-4*H*-1,4a-propano-2*H-*phenanthro[1,2-c]pyran-7-carboxylic acid,
or a pharmaceutically acceptable salt or hydrate thereof,
for use in a method of treating a fungal osteo-articular infection in a subject in need thereof, wherein the fungal osteo-articular infection is osteomyelitis, spondylodiscitis or arthritis. The fungal infection may be a yeast or mold infection that occurs in osteo-articular structures and that requires long-term antifungal therapy (more than 4 weeks). The compound or the pharmaceutically acceptable salt or hydrate thereof may be administered orally. The compound or the pharmaceutically acceptable salt or hydrate thereof may be administered daily for more than 4 weeks, or daily for 12 or more weeks.

In other preferred embodiments, the present invention provides a compound of Formula (IIa) (herein referred to as SCY-078 or ibrexafungerp): 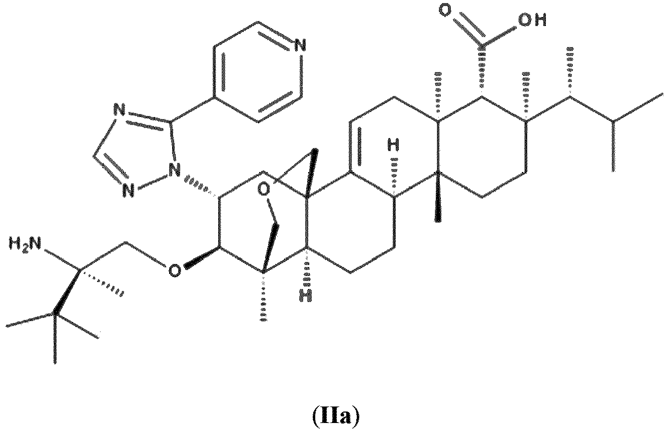 which is (1*S*,4a*R*,6a*S*,7*R*,8*R*,10a*R*,10b*R*,12a*R*,14*R*,15R)-15-[[(2*R*)-2-amino-2,3,3-trimethylbutyl]oxy]-8-[(1*R*)-1,2-dimethylpropyl]-14-[5-(4-pyridinyl)-1*H*-1,2,4-triazol-1-yl]-1,6,6a,7,8,9,10,10a,10b,11,12,12a-dodecahydro-1,6a,8,10a-tetramethyl-4*H*-1,4a-propano-*2H-*phenanthro[1,2-c]pyran-7-carboxylic acid,
or a pharmaceutically acceptable salt or hydrate thereof,
for use in a method of treating a fungal osteo-articular infection in a subject in need thereof, wherein the fungal osteo-articular infection is osteomyelitis, spondylodiscitis or arthritis. The fungal infection may be a yeast or mold infection that occurs in osteo-articular structures and that requires long-term antifungal therapy (more than 4 weeks). The compound or the pharmaceutically acceptable salt or hydrate thereof may be administered daily for more than 4 weeks, or daily for 12 or more weeks. Suitable dosing schedules include 750 mg of the compound of Formula (IIa) administered to the subject BID for 2 days, and then 750 mg of the compound of Formula (IIa) QD each subsequent day.

In preferred embodiments, the phosphate salt of a compound of Formula (II), or (IIa) is used or administered as described herein.

In preferred embodiments, the citrate salt of a compound of Formula (II), or (IIa) is used or administered as described herein.

The present invention also provides a pharmaceutical composition comprising a compound of Formula (II), or (IIa), or a pharmaceutically acceptable salt or hydrate thereof, and a pharmaceutically acceptable carrier, adjuvant, or vehicle, for use in the treatment of a fungal osteo-articular infection, wherein the fungal osteo-articular infection is osteomyelitis, spondylodiscitis or arthritis. The fungal infection may be a yeast or mold infection that occurs in osteo-articular structures and that requires long-term antifungal therapy (more than 4 weeks).

Also described herein, but not presently claimed, are methods of treating a fungal osteo-articular infection in a subject by administering a compound of Formula (I), or a pharmaceutically acceptable salt or hydrate thereof: 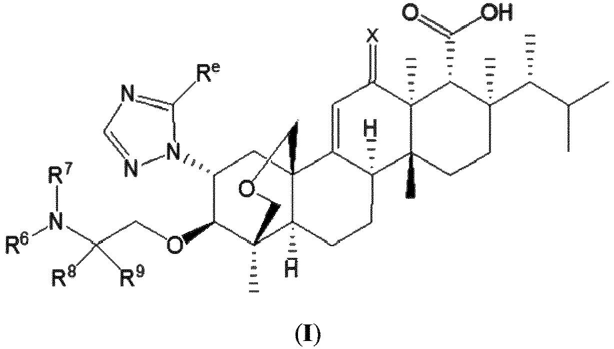 wherein:
X is O or H, H;
R^{e} is C(O)NR^{f}R^{g} or a 6-membered ring heteroaryl group containing 1 or 2 nitrogen atoms wherein the heteroaryl group is optionally mono-substituted on a ring carbon with fluoro or chloro or on a ring nitrogen with oxygen;
R^{f}, R^{g}, R⁶ and R⁷ are each independently hydrogen or C₁-C₃ alkyl;
R⁸ is C₁-C₄ alkyl, C₃-C₄ cycloalkyl or C₄-C₅ cycloalkyl-alkyl;
R⁹ is methyl or ethyl; and
R⁸ and R⁹ are optionally taken together to form a 6-membered saturated ring containing 1 oxygen atom. The fungal infection may be a yeast or mold infection that occurs in osteo-articular structures and that requires long-term antifungal therapy (more than 4 weeks). Infections treatable by these methods include but are not limited to osteomyelitis, spondylodiscitis and arthritis.

The compounds of Formula (I), (II), and (IIa), and pharmaceutically acceptable salts and/or hydrate forms thereof, have antimicrobial (*e.g*., antifungal) activities against yeasts and other fungi, including one or more of *Acremonium, Absidia* (*e.g., Absidia corymbifera*), *Alternaria, Aspergillus* (*e.g., Aspergillus clavatus, Aspergillus flavus, Aspergillus fumigatus, Aspergillus nidulans, Aspergillus niger, Aspergillus terreus,* and *Aspergillus versicolor*)*, Bipolaris, Blastomyces* (*e.g., Blastomyces dermatitidis*)*, Blastoschizomyces* (*e.g., Blastoschizomyces capitatus*)*, Candida* (*e.g., Candida albicans, Candida auris, Candida glabrata, Candida guilliermondii, Candida kefyr, Candida krusei, Candida lusitaniae, Candida parapsilosis, Candida pseudotropicalis, Candida stellatoidea, Candida tropicalis, Candida utilis, Candida lipolytica, Candida famata* and *Candida rugosa*)*, Cladosporium* (*e.g., Cladosporium carrionii* and *Cladosporium trichloides*)*, Coccidioides* (*e.g., Coccidioides immitis*)*, Cryptococcus* (*e.g., Cryptococcus neoformans*), *Curvularia, Cunninghamella* (*e.g., Cunninghamella elegans*)*, Dermatophyte, Exophiala* (*e.g., Exophiala dermatitidis* and *Exophiala spinifera*), *Epidermophyton* (*e.g., Epidermophyton floccosum*)*, Fonsecaea* (*e.g., Fonsecaea pedrosoi*), *Fusarium* (*e.g., Fusarium solani*)*, Geotrichum* (*e.g., Geotrichum candidum* and *Geotrichum clavatum*), *Histoplasma* (*e.g., Histoplasma capsulatum var. capsulatum*)*, Malassezia* (*e.g., Malassezia, furfur*), *Microsporum* (*e.g., Microsporum canis* and *Microsporum gypseum*)*, Mucor, Paracoccidioides* (*e.g., Paracoccidioides brasiliensis), Penicillium* (*e.g., Penicillium marneffei*), *Phialophora, Pityrosporum ovale, Pneumocystis* (*e.g., Pneumocystis carinii*)*, Pseudallescheria* (*e.g., Pseudallescheria boydii*)*, Rhizopus* (*e.g., Rhizopus microsporus var. rhizopodiformis* and *Rhizopus oryzae*)*, Saccharomyces* (*e.g., Saccharomyces cerevisiae*)*, Scedosporium* (*e.g., Scedosporium apiosperum*)*, Scopulariopsis, Sporothrix* (*e.g., Sporothrix schenckii*)*, Trichoderma, Trichophyton* (*e.g., Trichophyton mentagrophytes* and *Trichophyton rubrum*)*,* and *Trichosporon* (*e.g., Trichosporon asahii, Trichosporon beigelii,* and *Trichosporon cutaneum*)*.* The compounds are particularly effective against *Candida* species *and Aspergillus* species.

The compounds of Formula (I), (II), and (IIa), and pharmaceutically acceptable salts and/or hydrate forms thereof, can be made according to the synthesis methods disclosed in U.S. Patent No. 8,188,085.

As used herein, the term "alkyl" refers to any linear or branched chain alkyl group having a number of carbon atoms in the specified range. Thus, for example, "C₁₋₆ alkyl" (or "C₁-C₆ alkyl") refers to all of the hexyl alkyl and pentyl alkyl isomers as well as n-, iso-, sec- and t-butyl, n- and isopropyl, ethyl and methyl. As another example, "C₁₋₄ alkyl" refers to n-, iso-, sec- and t-butyl, n- and isopropyl, ethyl and methyl.

The term "cycloalkyl" refers to any cyclic ring of an alkane having a number of carbon atoms in the specified range. Thus, for example, "C₃₋₄ cycloalkyl" (or "C₃-C₄ cycloalkyl") refers to cyclopropyl and cyclobutyl.

The term "cycloalkyl-alkyl" (or equivalently "alkyl-cycloalkyl") as used herein refers to a system that includes an alkyl portion as described above and also includes a cycloalkyl portion as described above. Attachment to a "cycloalkyl-alkyl" (or "alkyl-cycloalkyl") may be through either the cycloalkyl or the alkyl portion. The specified number of carbon atoms in "cycloalkyl-alkyl" systems refers to the total number of carbon atoms in both the alkyl and the cycloalkyl parts. Examples of C₄-C₅ cycloalkyl-alkyl include but are not limited to methylcyclopropyl, dimethylcyclopropyl, methylcyclobutyl, ethylcyclopropyl, cyclopropylmethyl, cyclopropylethyl and cyclobutylmethyl.

The term "halogen" (or "halo") refers to fluorine, chlorine, bromine and iodine (alternatively referred to as fluoro, chloro, bromo, and iodo).

The term "or" as used herein denotes alternatives that may, where appropriate, be combined.

Unless expressly stated to the contrary, all ranges cited herein are inclusive. For example, a heterocyclic ring described as containing from "1 to 4 heteroatoms" means the ring can contain 1, 2, 3, or 4 heteroatoms. It is also to be understood that any range cited herein includes within its scope all of the sub-ranges within that range. Thus, for example, a heterocyclic ring described as containing from "1 to 4 heteroatoms" is intended to include as aspects thereof, heterocyclic rings containing 2 to 4 heteroatoms, 3 or 4 heteroatoms, 1 to 3 heteroatoms, 2 or 3 heteroatoms, 1 or 2 heteroatoms, 1 heteroatom, 2 heteroatoms, and so forth.

Any of the various cycloalkyl and heterocyclic/heteroaryl rings and ring systems defined herein may be attached to the rest of the compound at any ring atom (i.e., any carbon atom or any heteroatom) provided that a stable compound results. Suitable 5- or 6-membered heteroaromatic rings include, but are not limited to, pyridyl, pyrazinyl, pyrimidinyl, pyridazinyl and triazolyl.

A "stable" compound is a compound that can be prepared and isolated and whose structure and properties remain or can be caused to remain essentially unchanged for a period of time sufficient to allow use of the compound for the purposes described herein (*e.g*., therapeutic or prophylactic administration to a subject). Reference to a compound also includes stable complexes of the compound such as a stable hydrate.

As a result of the selection of substituents and substituent patterns, certain of the compounds of Formula (I), (II), and (IIa) can have asymmetric centers and can occur as mixtures of stereoisomers, or as individual diastereomers, or enantiomers. Unless otherwise indicated, all isomeric forms of these compounds (and pharmaceutically acceptable salts and/or hydrate forms thereof), whether isolated or in mixtures, are within the scope of the present invention. Also included within the scope of the present invention are tautomeric forms of the compounds as depicted (and pharmaceutically acceptable salts and/or hydrate forms thereof).

When any variable occurs more than one time in any constituent or in Formula (I), (II), or (IIa), its definition on each occurrence is independent of its definition at every other occurrence. Also, combinations of substituents and/or variables are permissible only if such combinations result in stable compounds.

The term "substituted" includes mono- and poly-substitution by a named substituent to the extent such single and multiple substitution (including multiple substitution at the same site) is chemically allowed. Unless expressly stated to the contrary, substitution by a named substituent is permitted on any atom in a ring (*e.g*., an aryl, a cycloalkyl, a heteroaryl, or a heterocyclyl) provided such ring substitution is chemically allowed and results in a stable compound.

A bond terminated by a wavy line is used herein to signify the point of attachment of a substituent group or partial structure. This usage is illustrated by the following example:

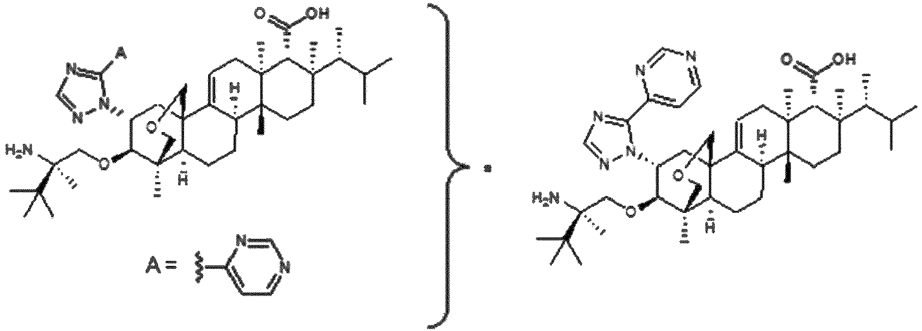

The compounds of Formula (I), (II), and (IIa), and pharmaceutically acceptable salts and/or hydrate forms thereof, are also useful in the preparation and execution of screening assays for antifungal compounds. For example, the compounds are useful for isolating mutants, which are excellent screening tools for identifying further antifungal compounds.

The compounds of Formula (I), (II), and (IIa) may be administered in the form of "pharmaceutically acceptable salts" or hydrates as appropriate. Other salts may, however, be useful in the preparation of the compounds or of their pharmaceutically acceptable salts. For example, when the compounds contain a basic amine group, they may be conveniently isolated as trifluoroacetic acid salts (*e.g*., following HPLC purification). Conversion of the trifluoroacetic acid salts to other salts, including pharmaceutically acceptable salts, may be accomplished by a number of standard methods known in the art. For example, an appropriate ion exchange resin may be employed to generate the desired salt. Alternatively, conversion of a trifluoroacetic acid salt to the parent free amine may be accomplished by standard methods known in the art (*e.g*., neutralization with an appropriate inorganic base such as NaHCO₃). Other desired amine salts may then be prepared in a conventional manner by reacting the free base with a suitable organic or inorganic acid. Representative pharmaceutically acceptable quaternary ammonium salts include the following: hydrochloride, sulfate, phosphate, carbonate, acetate, tartrate, citrate, malate, succinate, lactate, stearate, fumarate, hippurate, maleate, gluconate, ascorbate, adipate, gluceptate, glutamate, glucoronate, propionate, benzoate, mesylate, tosylate, oleate, lactobionate, laurylsulfate, besylate, caprylate, isetionate, gentisate, malonate, napsylate, edisylate, pamoate, xinafoate, napadisylate, hydrobromide, nitrate, oxalate, cinnamate, mandelate, undecylenate, and camsylate. Many of the compounds of Formula (I), (Ia), (II), and (IIa) carry an acidic carboxylic acid moiety, in which case suitable pharmaceutically acceptable salts thereof may include alkali metal salts, *e.g*., sodium or potassium salts; alkaline earth metal salts, *e.g*., calcium or magnesium salts; and salts formed with suitable organic ligands, *e.g*., quaternary ammonium salts.

Prodrugs of Formula (I), (II), and (IIa) may be used. In general, such prodrugs will be functional derivatives of the compounds, which are readily convertible in vivo into the required compound. Thus, the term "administering" shall encompass the treatment of the various conditions described with the compound specifically disclosed or with a compound that converts to the specified compound *in vivo* after administration to the patient. Conventional procedures for the selection and preparation of suitable prodrug derivatives are described, for example, in "Design of Prodrugs," ed. H. Bundgaard, Elsevier, 1985. Metabolites of the compounds of Formula (I), (II), and (IIa) include active species produced upon introduction of the compounds into the biological milieu.

The term "administration" and variants thereof (*e.g*., "administering" a compound) mean providing a compound (optionally in the form of a salt or hydrate thereof) or a prodrug of the compound to the subject in need of treatment. When a compound of Formula (I), (II), and (IIa) or pharmaceutically acceptable salt thereof or a hydrate or prodrug thereof is provided in combination with a second active agent (*e.g*., other antifungal and/or antibacterial agents useful for treating fungal and/or bacterial infections), "administration" and its variants are each understood to include concurrent and sequential provision of the compound (or the salt, hydrate, or prodrug thereof) and of the other active agent.

As used herein, the term "composition" is intended to encompass a product comprising the specified ingredients, as well as any product that results, directly or indirectly, from combining the specified ingredients.

By "pharmaceutically acceptable" is meant that the ingredients of the pharmaceutical composition must be compatible with each other and not deleterious to the recipient thereof.

The term "subject" (alternatively referred to herein as "patient") as used herein refers to an animal, preferably a mammal, most preferably a human, who has been the object of treatment, observation, or experiment.

The term "effective amount" as used herein means an amount of active ingredient or pharmaceutical agent that elicits the biological or medicinal response in a tissue, system, animal, or human that is being sought by a researcher, veterinarian, medical doctor, or other clinician. In one embodiment, the "effective amount" can be a therapeutically effective amount that alleviates the symptoms of the disease or condition being treated. In another embodiment, the "effective amount" can be a prophylactically effective amount for prophylaxis of the symptoms of the disease or condition being prevented or for reducing the likelihood of occurrence. The term can also refer to an inhibition effective amount of the enfumafungin derivative sufficient to inhibit (1,3)-β-D-glucan synthase and thereby elicit the response being sought.

References to "treat," "treating," "treatment," and variants thereof, generally refer to a treatment that, after it is administered, results in resolution or improvement of one or more signs or symptoms associated with a fungal infection, or that results in eradication of the fungi responsible for an infection, or any combination of these outcomes.

For the purpose of treating a fungal infection, the compound of Formula (I), (II), or (IIa) (optionally in the form of a salt or a hydrate) can be administered in conventional ways available for use in conjunction with pharmaceuticals.

For the purpose of treating a fungal infection, the compound of Formula (I), (II), or (IIa) (optionally in the form of a salt or a hydrate) can be administered alone as an individual therapeutic agent or with one or more other antifungal agents (sequentially or concurrently) as a combination of therapeutic agents.

For the purpose of treating a fungal infection, the compound of Formula (I), (II), or (IIa) (optionally in the form of a salt or a hydrate) can be administered with a pharmaceutical carrier selected on the basis of the chosen route of administration and standard pharmaceutical practice.

For example, the compounds of Formula (I), (II), and (IIa), and pharmaceutically salts and/or hydrate forms thereof, can be administered by one or more of the following routes: orally, parenterally (including subcutaneous injections, intravenous, intramuscular, intra-lesion injection or infusion techniques), by inhalation (*e.g*., nasal or buccal inhalation spray, aerosols from metered dose inhalator, and dry powder inhalator), by nebulizer, ocularly, topically, transdermally, or rectally, in the form of a unit dosage of a pharmaceutical composition containing an effective amount of the compound and conventional non-toxic pharmaceutically-acceptable carriers, adjuvants and vehicles. Liquid preparations suitable for oral administration (*e.g*., suspensions, syrups, elixirs and the like) can be prepared according to techniques known in the art and can employ the usual media such as water, glycols, oils, alcohols and the like. Solid preparations suitable for oral administration (*e.g*., powders, pills, capsules and tablets) can be prepared according to techniques known in the art and can employ such solid excipients as starches, sugars, kaolin, lubricants, binders, disintegrating agents and the like. Parenteral compositions can be prepared according to techniques known in the art and typically employ sterile water as a carrier and optionally other ingredients, such as a solubility aid. Injectable solutions can be prepared according to methods known in the art wherein the carrier comprises a saline solution, a glucose solution or a solution containing a mixture of saline and glucose.

Further description of methods suitable for use in preparing pharmaceutical compositions and of ingredients suitable for use in said compositions is provided in Remington's Pharmaceutical Sciences, 20th edition, edited by A. R. Gennaro, Mack Publishing Co., 2000.

The compounds of Formula (I), (II), and (IIa), and pharmaceutically acceptable salts and/or hydrate forms thereof, can be administered, *e.g*., orally or intravenously, in a dosage range of, for example, 0.001 to 1000 mg/kg of mammal (*e.g*., human) body weight per day in a single dose or in divided doses. An example of a dosage range is 0.01 to 500 mg/kg body weight per day orally or intravenously in a single dose or in divided doses. Another example of a dosage range is 0.1 to 50 mg/kg body weight per day orally or intravenously in single or divided doses. For oral administration, the compositions can be provided in the form of tablets or capsules containing, for example, 1.0 to 1000 milligrams of the active ingredient, particularly 1, 5, 10, 15, 20, 25, 50, 75, 100, 150, 200, 250, 300, 400, 500, 600, 750, and 1000 milligrams of the active ingredient for the symptomatic adjustment of the dosage to the patient to be treated. The specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy. For example, in embodiments, a pharmaceutically acceptable salt of the compound of Formula (IIa) is administered to a subject to provide a total daily dose of 250 to 1500 mg of the compound of Formula (IIa). In certain embodiments, a total daily dose of 250 mg, or a total daily dose of 500 mg, or a total daily dose of 750 mg, or a total daily dose of 1000 mg, or a total daily dose of 1500 mg of the compound of Formula (IIa) is administered; the total daily dose may be administered on a once-daily basis or it may be divided such as for BID (twice daily) dosing or TID (thrice daily) dosing. In embodiments, a pharmaceutically acceptable salt of the compound of Formula (IIa) is administered QD or BID to provide 250 to 750 mg of the compound of Formula (IIa) per day.

Described herein, but not presently claimed, are methods for treating a osteo-articular fungal infection, comprising administering an effective amount of a compound of Formula (I), (II), or (IIa) (or a pharmaceutically acceptable salt or hydrate thereof).

Antifungal activity of compounds can be demonstrated by various assays known in the art, for example, by their minimum inhibitory concentration (MIC) against yeasts and minimum effective concentration (MEC) against filamentous molds and dermatophytes in a broth microdilution assay, or in vivo evaluation of the anti*-Candida* and anti*-Aspergillus* activity in mouse or rabbit models. The compounds of Formula (I) provided in the Examples of U.S. Patent No. 8,188,085 were generally found to inhibit the growth of *Candida* spp. in the range of <0.03-32 µg/mL or to give an MEC against *Aspergillus fumigatus* in the range of <0.03-32 µg/mL.

### EXAMPLES

The following examples serve only to illustrate the invention and its practice. The examples are not to be construed as limitations on the scope or spirit of the invention.

### EXAMPLE 1

### Evaluation of the clinical efficacy of orally administered SCY-078 for the treatment of osteo-articular fungal infections

This example presents treatment courses of two patients with *Candida* spondylodiscitis who were enrolled in an "Open-Label Study to Evaluate Efficacy and Safety of SCY-078 in Patients With Refractory or Intolerant Fungal Diseases (FURI)" (clinicaltrials.gov identifier NCT03059992).

**Objective:** The objective of the study is to evaluate the efficacy of orally administered SCY-078 for the treatment of fungal infections that are refractory to or intolerant of approved antifungal agents.

**Study design:** This is a multicenter, open-label, non-comparator, single-arm study to evaluate the efficacy, safety, and pharmacokinetics (PK) of SCY-078 (administered as the citrate salt) in male and female subjects ≥18 years of age with documented invasive and/or severe candidiasis that has been refractory to or intolerant of, or has shown toxicities associated with standard of care (SoC) antifungal treatment. Subjects must have a documented eligible acute or chronic invasive candidiasis (including candidemia) and meet all study criteria to be considered for enrollment. Eligible subjects must also have documented evidence of failure of, intolerance to, or toxicity associated with a currently approved SoC antifungal treatment. Subjects are also eligible if, in the judgement of the investigator, continued intravenous antifungal therapy is not feasible or desirable due to clinical or logistical circumstances or if other oral antifungal alternatives are not appropriate. Inclusion of each subject in the study must be approved by the sponsor prior to initiation of study drug. Eligible subjects will receive an initial loading dose of 750 mg of SCY-078 (3 tablets of 250 mg) twice a day (BID) during the first 2 days of treatment and then subsequent oral doses of 750 mg once a day (QD). There will be 1 Screening visit, 1 Baseline visit (also considered Treatment Day 1), 2 additional scheduled Treatment visits (Treatment Days 3 to 5 and 7 to 10) and treatment visits every 14 days thereafter (for up to a total of 90 days), 1 follow-up visit 6 weeks after end of treatment (EoT) (Week 6 Follow up), and 2 survival visits/contacts.

**Efficacy:** Efficacy will be assessed primarily in terms of global success (both clinical success and mycological success). Complete global response, partial global response, clinical success, and mycological success will also be assessed. The primary time point for the determination of efficacy is EoT. The oral formulation of SCY-078 used in this study is compressed tablets containing the citrate salt of SCY-078. Excipients in the tablet include silicified microcrystalline cellulose, mannitol, crospovidone, colloidal silicon dioxide, magnesium stearate, and butylated hydroxyanisole. Each tablet provides 250 mg of SCY-078 active ingredient on a free base basis.

**Results:** Two cases from this study of patients with osteo-articular fungal infection are reported below.

Example A: A 50-year-old man with a diagnosis of acute myeloid leukemia (AML), who previously had an allogeneic stem cell transplant (SCT) and graft-versus-host disease (GVHD), developed *Candida albicans* spondylodiscitis at L4/L5. Five months earlier, the patient had two episodes of *Candida albicans* candidemia, both with prolonged fungemia for 4 and 7 days, respectively. The patient was enrolled in the FURI study and treated with oral ibrexafungerp (750 mg BID for 2 days, followed by 750 mg QD each subsequent day). Posterior lumbar interbody fusion (PLIF) at L4/L5 was performed. At the time of evaluation, the patient had been treated with oral ibrexafungerp for 277 days. Evaluation of the patient's response to date was graded as improved, due to reduction of pain, defervescence and improvement of mobility. Possible study drug-related adverse events were diarrhea, flatulence and nausea.

Example B: A 58-year-old man with relapsed bladder cancer after radical cystectomy with ileal conduit developed *Candida tropicalis* spondylodiscitis at L5/S1. Three months earlier, he had *Candida tropicalis* fungemia. The patient was enrolled in the FURI study and treated with oral ibrexafungerp (750 mg BID for 2 days, followed by 750 mg QD each subsequent day). At the time of evaluation, the patient had been treated with oral ibrexafungerp for 88 days. Evaluation of the patient's response to date was graded as resolved, due to resolution of clinical symptoms. Possible study drug-related adverse event was diarrhea.

While this invention has been particularly shown and described with references to preferred embodiments thereof, it will be understood in light of the present disclosure by those skilled in the art that various changes in form and details may be made therein without departing from the scope of the invention encompassed by the appended claims.

## Claims

1. A compound of Formula (II): 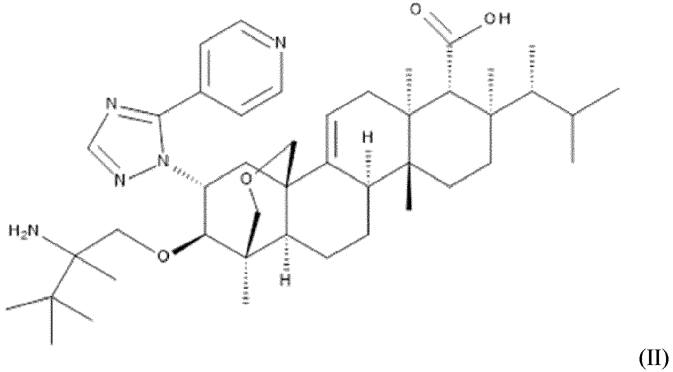 which is (1*S*,4a*R*,6a*S*,7*R*,8*R*,10a*R*,10b*R*,12a*R*,14*R*,*15R*)-15-[[2-amino-2,3,3-trimethylbutyl]oxy]-8-[(1*R*)-1,2-dimethylpropyl]-14-[5-(4-pyridinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid,
or a pharmaceutically acceptable salt or hydrate thereof,
for use in a method of treating a fungal osteo-articular infection in a subject in need thereof, wherein the fungal osteo-articular infection is osteomyelitis, spondylodiscitis, or arthritis.

2. The compound, or pharmaceutically acceptable salt or hydrate thereof, for use according to claim 1, wherein the compound or the pharmaceutically acceptable salt or hydrate thereof is administered orally.

3. The compound, or pharmaceutically acceptable salt or hydrate thereof, for use according to claim 1, wherein the fungal osteo-articular infection is caused by *Candida* spp.

4. The compound, or pharmaceutically acceptable salt or hydrate thereof, for use according to claim 1, wherein the fungal osteo-articular infection is caused by *Aspergillosis* spp.

5. The compound, or pharmaceutically acceptable salt or hydrate thereof, for use according to any one of claims 1-4, wherein the compound or the pharmaceutically acceptable salt or hydrate thereof is administered daily for more than 4 weeks.

6. The compound, or pharmaceutically acceptable salt or hydrate thereof, for use according to any one of claims 1-4, wherein the compound or the pharmaceutically acceptable salt or hydrate thereof is administered daily for 12 or more weeks.

7. The compound, or pharmaceutically acceptable salt or hydrate thereof, for use according to any one of claims 1-6, wherein the subject is a human.

8. A compound of Formula (IIa): 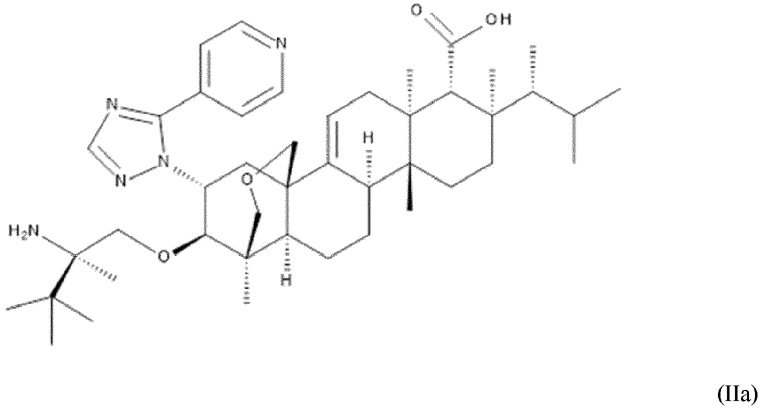 which is (1*S*,4a*R*,6a*S*,7*R*,8*R*,10a*R*,10b*R*,12a*R*,14*R*,15*R*)-15-[[(2*R*)-2-amino-2,3,3-trimethylbutyl]oxy]-8-[(1*R*)-1,2-dimethylpropyl]-14-[5-(4-pyridinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid,
or a pharmaceutically acceptable salt or hydrate thereof,
for use in a method of treating a fungal osteo-articular infection in a subject in need thereof, the method comprising orally administering to the subject the compound of Formula (IIa) or the pharmaceutically acceptable salt or hydrate thereof,
wherein the fungal osteo-articular infection is osteomyelitis, spondylodiscitis, or arthritis.

9. The compound, or pharmaceutically acceptable salt or hydrate thereof, for use according to claim 8, wherein the compound or the pharmaceutically acceptable salt or hydrate thereof is administered daily for more than 4 weeks.

10. The compound, or pharmaceutically acceptable salt or hydrate thereof, for use according to claim 8, wherein 750 mg of the compound of Formula (IIa) is administered BID for 2 days, and then 750 mg of the compound of Formula (IIa) is administered QD each subsequent day.

11. The compound, or pharmaceutically acceptable salt or hydrate thereof, for use according to claim 9, wherein 750 mg of the compound of Formula (IIa) is administered BID for 2 days, and then 750 mg of the compound of Formula (IIa) is administered QD each subsequent day.

## Patentansprüche

1. Verbindung der Formel (II): 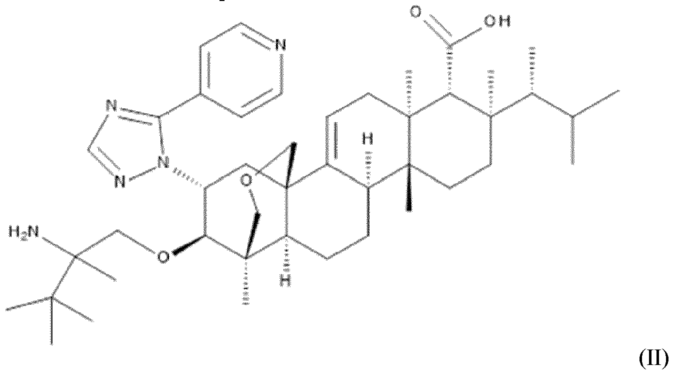
die (1*S*,4a*R*,6a*S*,7*R*,8*R*,10a*R*,10b*R*,12a*R*,14*R*,15*R*)-15-[[2-Amino-2,3,3-trimethylbutyl]oxy]-8-[(1*R*)-1,2-dimethylpropyl]-14-[5-(4-pyridinyl)-1*H*-1,2,4-triazol-1-yl]-1,6,6a,7,8,9,10,10a,10b,11,12,12a-dodecahydro-1,6a,8,10a-tetramethyl-4H-1,4a-propano-2H-phenanthro[1,2-c]pyran-7-carbonsäure ist,
oder ein pharmazeutisch annehmbares Salz oder Hydrat davon,
zur Verwendung bei einem Verfahren zur Behandlung einer fungiellen osteoartikulären Infektion bei einem Subjekt mit Bedarf daran, wobei die fungielle osteoartikuläre Infektion Osteomyelitis, Spondylodiszitis oder Arthritis ist.

2. Verbindung oder pharmazeutisch annehmbares Salz oder Hydrat davon zur Verwendung gemäß Anspruch 1, wobei die Verbindung oder das pharmazeutisch annehmbare Salz oder Hydrat davon oral verabreicht wird.

3. Verbindung oder pharmazeutisch annehmbares Salz oder Hydrat davon zur Verwendung gemäß Anspruch 1, wobei die fungielle osteoartikuläre Infektion von *Candida* spp. verursacht ist.

4. Verbindung oder pharmazeutisch annehmbares Salz oder Hydrat davon zur Verwendung gemäß Anspruch 1, wobei die fungielle osteoartikuläre Infektion von *Aspergillosis* spp. verursacht ist.

5. Verbindung oder pharmazeutisch annehmbares Salz oder Hydrat davon zur Verwendung gemäß einem der Ansprüche 1-4, wobei die Verbindung oder das pharmazeutisch annehmbare Salz oder Hydrat davon täglich für mehr als 4 Wochen verabreicht wird.

6. Verbindung oder pharmazeutisch annehmbares Salz oder Hydrat davon zur Verwendung gemäß einem der Ansprüche 1-4, wobei die Verbindung oder das pharmazeutisch annehmbare Salz oder Hydrat davon täglich für 12 oder mehr Wochen verabreicht wird.

7. Verbindung oder pharmazeutisch annehmbares Salz oder Hydrat davon zur Verwendung gemäß einem der Ansprüche 1-6, wobei das Subjekt ein Mensch ist.

8. Verbindung gemäß Formel (IIa): 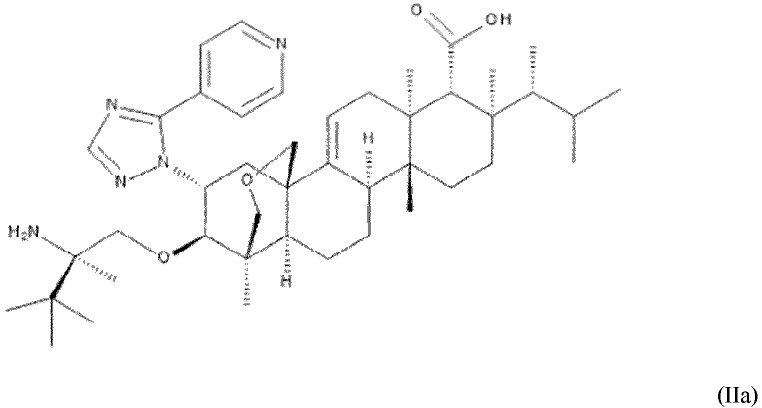
die (1*S*,4a*R,*6a*S*,7*R*,8*R*,10a*R*,10b*R*,12a*R*,14*R*,15*R*)-15-[[(2*R*)-2-Amino-2,3,3-trimethylbutyl]oxy]-8-[(1*R*)-1,2-dimethylpropyl]-14-[5-(4-pyridinyl)-1*H*-1,2,4-triazol-1-yl]-1, 6, 6a, 7,8,9,10,10a,10b,11,12,12a-dodecahydro-1,6a,8,10a-tetramethyl-4*H*-1,4a-propano-2*H-*phenanthro[1,2-c]pyran-7-carbonsäure ist,
oder ein pharmazeutisch annehmbares Salz oder Hydrat davon,
zur Verwendung bei einem Verfahren zur Behandlung einer fungiellen osteoartikulären Infektion bei einem Subjekt mit Bedarf daran, wobei das Verfahren orales Verabreichen der Verbindung der Formel (IIa) oder des pharmazeutisch annehmbaren Salzes oder Hydrats davon an das Subjekt umfasst,
wobei die fungielle osteoartikuläre Infektion Osteomyelitis, Spondylodiszitis oder Arthritis ist.

9. Verbindung oder pharmazeutisch annehmbares Salz oder Hydrat davon zur Verwendung gemäß Anspruch 8, wobei die Verbindung oder das pharmazeutisch annehmbare Salz oder Hydrat davon täglich für mehr als 4 Wochen verabreicht wird.

10. Verbindung oder pharmazeutisch annehmbares Salz oder Hydrat davon zur Verwendung gemäß Anspruch 8, wobei 750 mg der Verbindung der Formel (IIa) BID für 2 Tage verabreicht werden und dann 750 mg der Verbindung der Formel (IIa) jeden nachfolgenden Tag QD verabreicht werden.

11. Verbindung oder pharmazeutisch annehmbares Salz oder Hydrat davon zur Verwendung gemäß Anspruch 9, wobei 750 mg der Verbindung der Formel (IIa) BID für 2 Tage verabreicht werden und dann 750 mg der Verbindung der Formel (IIa) jeden nachfolgenden Tag QD verabreicht werden.

## Revendications

1. Composé de formule (II) : 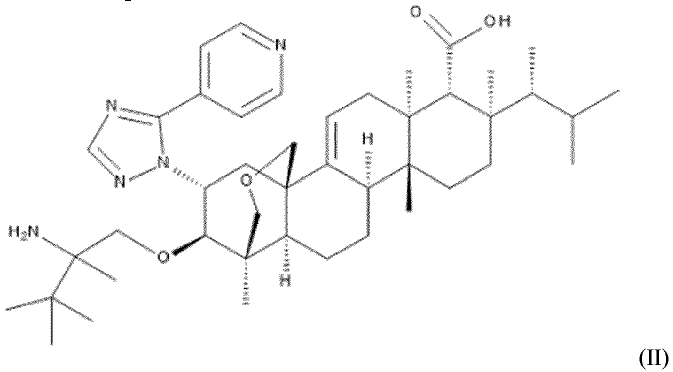
qui est l'acide (1*S*,4a*R*,6a*S*,7*R*,8*R*,10a*R*,10b*R*,12a*R*,14*R*,15*R*)-15-[[2-amino-2,3,3-triméthylbutyl]oxy]-8-[(1R)-1,2-diméthylpropyl]-14-[5-(4-pyridinyl)-1*H*-1,2,4-tnazol-1-yl]-1,6,6a,7,8,9,10,10a,10b,11,12,12a-dodécahydro-1,6a,8,10a-tétraméthyl-4*H*-1,4a-propano-2*H-*phénanthro[1,2-c]pyran-7-carboxylique,
ou un sel ou un hydrate pharmaceutiquement acceptable correspondant,
pour une utilisation dans un procédé de traitement d'une infection ostéoarticulaire fongique chez un sujet qui en a besoin, dans lequel l'infection ostéoarticulaire fongique est une ostéomyélite, une spondylodiscite ou une arthrite.

2. Composé, ou sel ou hydrate pharmaceutiquement acceptable correspondant, pour une utilisation selon la revendication 1, dans lequel le composé ou le sel ou l'hydrate pharmaceutiquement acceptable correspondant est administré par voie orale.

3. Composé, ou sel ou hydrate pharmaceutiquement acceptable correspondant, pour une utilisation selon la revendication 1, dans lequel l'infection fongique ostéoarticulaire est causée par *Candida* spp.

4. Composé, ou sel ou hydrate pharmaceutiquement acceptable correspondant, pour une utilisation selon la revendication 1, dans lequel l'infection fongique ostéoarticulaire est causée par Aspergillus spp.

5. Composé, ou sel ou hydrate pharmaceutiquement acceptable correspondant, pour une utilisation selon l'une quelconque des revendications 1-4, dans lequel le composé ou le sel ou l'hydrate pharmaceutiquement acceptable correspondant est administré quotidiennement pendant plus de 4 semaines.

6. Composé, ou sel ou hydrate pharmaceutiquement acceptable correspondant, pour une utilisation selon l'une quelconque des revendications 1-4, dans lequel le composé ou le sel ou l'hydrate pharmaceutiquement acceptable correspondant est administré quotidiennement pendant 12 semaines ou plus.

7. Composé, ou sel ou hydrate pharmaceutiquement acceptable correspondant, pour une utilisation selon l'une quelconque des revendications 1-6, dans lequel le sujet est un humain.

8. Composé de formule (IIa) : 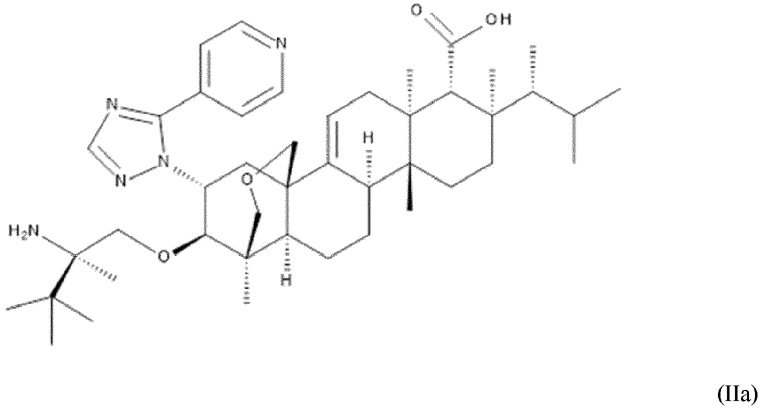
qui est l'acide (1*S*,4a*R*,6a*S*,7*R*,8*R*,10a*R*,10b*R*,12a*R*,14*R*,15*R*)-15-[[(2*R*)-2-amino-2,3,3-triméthylbutyl]oxy]-8-[(1*R*)-1,2-diméthylpropyl]-14-[5-(4-pyridinyl)-*1H*-1,2,4-triazol-1-yl]-1,6,6a,7,8,9,10,10a,10b,11,12,12a-dodécahydro-1,6a,8,10a-tétraméthyl-*4H*-1,4a-propano-*2H-*phénanthro[1,2-c]pyran-7-carboxylique,
ou un sel ou un hydrate pharmaceutiquement acceptable correspondant,
pour une utilisation dans un procédé de traitement d'une infection ostéoarticulaire fongique chez un sujet qui en a besoin, le procédé comprenant une administration par voie orale au sujet du composé de formule (IIa) ou du sel ou de l'hydrate pharmaceutiquement acceptable correspondant,
dans lequel l'infection ostéoarticulaire fongique est une ostéomyélite, une spondylodiscite ou une arthrite.

9. Composé, ou sel ou hydrate pharmaceutiquement acceptable correspondant, pour une utilisation selon la revendication 8, dans lequel le composé ou le sel ou l'hydrate pharmaceutiquement acceptable correspondant est administré quotidiennement pendant plus de 4 semaines.

10. Composé, ou sel ou hydrate pharmaceutiquement acceptable correspondant, pour une utilisation selon la revendication 8, dans lequel 750 mg du composé de formule (IIa) sont administrés deux fois par jour pendant 2 jours, puis 750 mg du composé de formule (IIa) sont administrés une fois par jour chaque jour subséquent.

11. Composé, ou sel ou hydrate pharmaceutiquement acceptable correspondant, pour une utilisation selon la revendication 9, dans lequel 750 mg du composé de formule (IIa) sont administrés deux fois par jour pendant 2 jours, et puis 750 mg du composé de formule (IIa) sont administrés une fois par jour chaque jour subséquent.
